# EUROPEAN PATENT APPLICATION

(11) **EP 4 736 918 A1**
(43) Date of publication of application: **06.05.2026**
(21) Application number: 25213143.8
(22) Date of filing: 03.11.2025
(51) Int. Cl.: A61M 16/04

(54) **INTUBATION TUBE HOLDER DEVICE**

(30) Priority: 04.11.2024 US 202418936537
(71) Applicant: Medline Industries, LP, Springfield, IL 62703 (US)
(72) Inventor: DREW, Douglas, Raleigh, NC 27614 (US); DWYER, Daniel P., Cary, NC 27519 (US); JACKSON, Christopher S., Raleigh, NC 27614 (US)
(74) Representative: Maiwald GmbH

(57) **Abstract**

An endotracheal tube holder device includes a head strap, a holding body, and a tube holder. The holding body includes a first cheek bracket and a second cheek bracket connected by a bar. The first cheek bracket includes a first strap loop and a second strap loop, and the second check bracket includes a third strap loop and a fourth strap loop. The tube holder is secured to the holding body and is configured to secure an endotracheal tube. The first strap loop, the second strap loop, the third strap loop, and the fourth strap loop are configured to secure the head strap to the holding body.

## Description

### CROSS-REFERENCE TO RELATED APPLICATIONS

Not applicable.

### STATEMENT REGARDING FEDERALLY SPONSORED RESEARCH

Not applicable.

### BACKGROUND

The present disclosure is generally directed to medical tube devices, such as may be used for intubation, and more particularly to a device for holding an endotracheal tube or other intubation device on a patient.

Intubation tubes, such as endotracheal tubes, are inserted into a patient for a variety of reasons. In the case of an endotracheal tube, the tube is inserted to establish and maintain a patient's airway during resuscitation, anesthesia, and other critical care procedures. Endotracheal tubes help deliver air to patients who cannot breathe on their own. They are often placed prior to surgery or are used for critically ill patients that require assistance breathing for extended periods of time. Once inserted, endotracheal tubes must be fastened in a fixed position to prevent movement of the endotracheal tube for periods of up to several days.

In order to properly secure the intubation tubes to the patient, known designs have disclosed a variety of securement components. However, there are a variety of deficiencies with these securement components. Thus, there is a need for an improved intubation tube holder system.

### SUMMARY OF THE DISCLOSURE

Some non-limiting examples of the disclosure provide an endotracheal tube holder device or an intubation tube holder system that includes a head strap, a holding body, and a tube holder. The holding body includes a first cheek bracket and a second cheek bracket connected by a bar. The first cheek bracket includes a first strap loop and a second strap loop, and the second cheek bracket includes a third strap loop and a fourth strap loop. The tube holder is secured to the holding body and is configured to secure an endotracheal tube. The first strap loop, the second strap loop, the third strap loop, and the fourth strap loop are configured to secure the head strap to the holding body.

In some non-limiting examples, the holding body further includes a first pad and a second pad that are configured to secure to a patients face.

In some non-limiting examples, the first pad and the second pad are transparent or translucent.

In some non-limiting examples, the first strap loop comprises a first axis, the second strap loop comprises a second axis, the third strap loop comprises a third axis, and the fourth strap loop comprises a fourth axis.

In some non-limiting examples, the first axis and the second axis form an angle that is between about 95° and about 170°.

In some non-limiting examples, the tube holder includes a tube holder body and a plurality of teeth that extend from a bottom surface of the tube holder body.

In some non-limiting examples, each of the plurality of teeth define an axis. Each of the axes of the plurality of teeth are substantially perpendicular to an outer surface of the endotracheal tube when the endotracheal tube is attached to the endotracheal tube holder device.

In some non-limiting examples, the tube holder comprises a bite block.

Some non-limiting examples of the disclosure provide a system. The system includes a holding body, a head strap secured to the holding body at four points of contact, and a tube holder secured with the holding body. The tube holder includes a tube holder body and a plurality of teeth that extend from a bottom surface of the tube holder body. The plurality of teeth includes a plurality of first teeth and a plurality of second teeth. A distance between the bottom surface of the tube holder body and a tip of one of the plurality of first teeth is larger than a distance between the bottom surface of the tube holder body and a tip of one of the plurality of second teeth.

In some non-limiting examples, the holding body further includes a first pad and a second pad that are configured to secure to a patients face.

In some non-limiting examples, the first pad and the second pad are transparent or translucent.

In some non-limiting examples, the holding body includes a first cheek bracket and a second cheek bracket. The first cheek bracket includes a first strap loop and a second strap loop, and the second cheek bracket includes a third strap loop and a fourth strap loop. The first strap loop, the second strap loop, the third strap loop, and the fourth strap loop define the four points of contact with the head strap.

In some non-limiting examples, the bottom surface of the tube holder body defines a longitudinal center plane that is normal to the bottom surface. Each of the plurality of teeth comprise an axis that is angled with respect to the longitudinal center plane.

In some non-limiting examples, a tube strap is secured with the tube holder and configured to wrap around an endotracheal tube. The tube strap comprises a plurality of ribs thereon.

In some non-limiting examples, the plurality of teeth are aligned in four columns. The plurality of teeth in one of the four columns coverage toward the plurality teeth in a different column below the bottom surface.

In some non-limiting examples, the endotracheal tube holder device includes more second teeth than first teeth.

Some non-limiting examples of the disclosure provide a system that includes a holding body and a tube holder. The holding body includes a first pad and a second pad. The first pad and the second pad are transparent or translucent. The tube holder is secured with the holding body. The tube holder includes a tube holder body and a plurality of teeth that extend from a bottom surface of the tube holder body. The bottom surface of the tube holder body defines a longitudinal center plane that is normal to the bottom surface. Each of the plurality of teeth comprise an axis that is not parallel with the longitudinal center plane.

In some non-limiting examples, each of the axes of the plurality of teeth converge toward the longitudinal center plane below the bottom surface.

In some non-limiting examples, the system further comprises a head strap secured to the holding body at four points of contact.

In some non-limiting examples, a tube strap is secured with the tube holder and configured to wrap around an endotracheal tube. The tube strap comprises a plurality of ribs thereon.

The foregoing and other aspects and advantages of the present disclosure will appear from the following description. In the description, reference is made to the accompanying drawings that form a part hereof, and in which there is shown by way of illustration one or more exemplary versions. These versions do not necessarily represent the full scope of the disclosure.

### BRIEF DESCRIPTION OF THE DRAWINGS

The following drawings are provided to help illustrate various features of non-limiting examples of the disclosure and are not intended to limit the scope of the disclosure or exclude alternative implementations.
FIG. 1 is a top, front, and right isometric view of a non-limiting example of a holder system according to aspects of the present disclosure.
FIG. 2 is a bottom, front, and left isometric view of the holder device of FIG. 1.
FIG. 3 is a front elevational view of the holder system of FIG. 1.
FIG. 4 is a rear elevational view of the holder system of FIG. 1.
FIG. 5 is a right side elevational view of the holder system of FIG. 1.
FIG. 6 is a left side elevational view of the holder system of FIG. 1.
FIG. 7 is a top plan view of the holder system of FIG. 1.
FIG. 8 is a bottom plan view of the holder system of FIG. 1.
FIG. 9 is a top, rear, and left isometric view of the holder of FIG. 1.
FIG. 10 is a front elevational view of the holder of FIG. 9.
FIG. 11 is a rear elevational view of the holder of FIG. 9.
FIG. 12 is a right side elevational view of the holder of FIG. 9.
FIG. 13 is a top plan view of the holder of FIG. 9.
FIG. 14 is a bottom plan view of the holder of FIG. 9.
FIG. 15 is a cross-sectional view of the holder system taken along plane 15-15 of FIG. 3 with a head strap removed for clarity.
FIG. 16 is a bottom isometric view of the holder system of FIG. 1 with the head strap removed for clarity.
FIG. 17 is a top isometric view of the holder system of FIG. 1 with the head strap and a tube holder removed for clarity.
FIG. 18 is an enlarged, partial cross-sectional view of the holder taken along plane 18-18 of FIG. 12.
FIG. 19 is a top, front, and right isometric view of the holder system of FIG. 1 holding, for example, an intubation tube.

### DETAILED DESCRIPTION OF THE PRESENT DISCLOSURE

The following discussion and accompanying figures disclose various embodiments or configurations of a holder system that includes a holding assembly, a head strap, and a plurality of additional components. Although embodiments of the holder system are disclosed with reference to securing or holding an intubation tube, concepts associated with embodiments of the holder system may be applied to a wide range of products. For example, the holder system may be used to secure or hold various types of tubes, medical devices, and products and is not limited to just holding or securing intubation tubes, including endotracheal tubes.

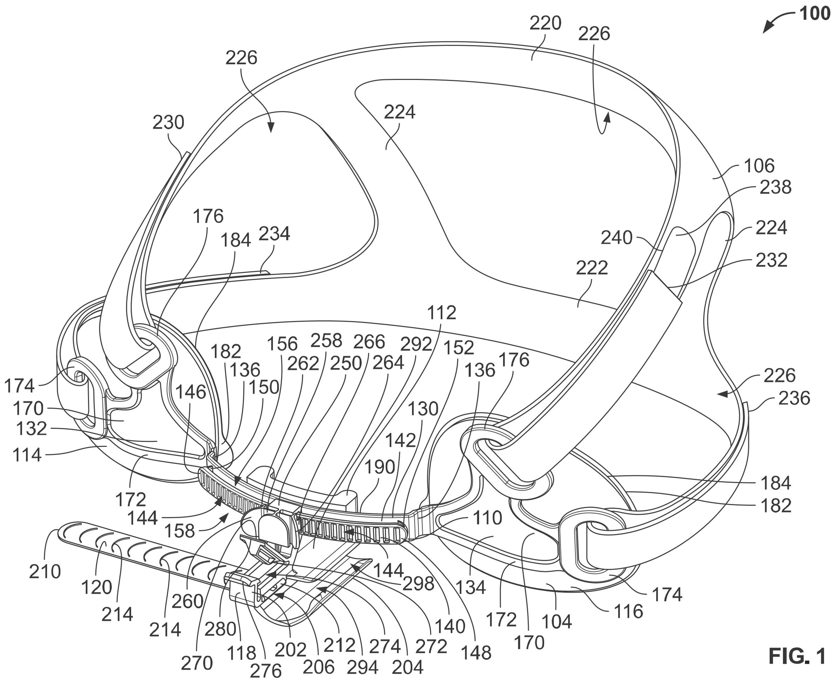

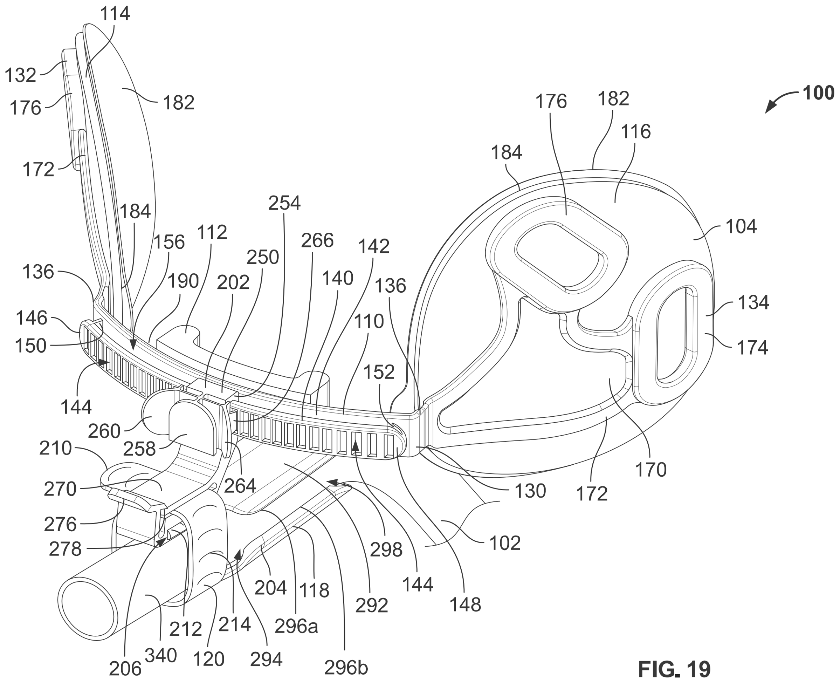

Referring now to the drawings, FIGS. 1-8 depict an intubation tube holder system 100 for securing or holding a tube 102 or other component (see FIG. 19). As will be described, the holder system 100 may be used to secure an endotracheal tube to a patient, as one non-limiting example. As such, the holder system 100 may be referred to as an endotracheal tube holder system. However, such referenced to the holder system 100 being an endotracheal tube holder system is in no way limiting of the system to securing only endotracheal tubes, as opposed to, for example, other tubes, devices, or components, such as may be needed to be secured to a patient's head or proximate to orifices, such as the mouth or nose. Thus, reference to a "tube holder" or "tube" or "endotracheal tube" are not limiting to the system 100 and other configurations as will be described herein.

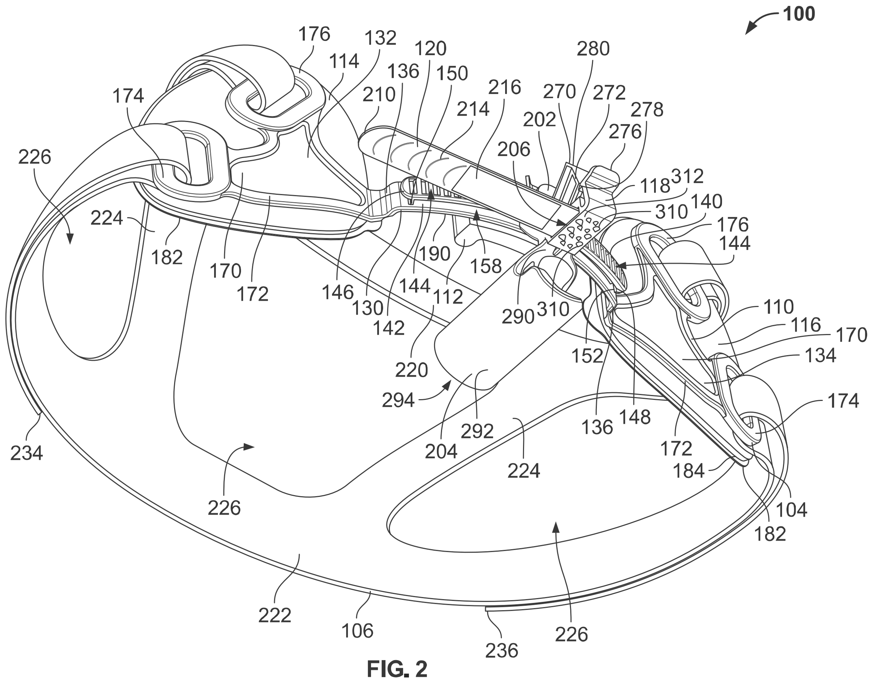

Referring specifically to FIGS. 1 and 2, the system 100 includes a holding assembly 104 and a head strap 106. The holding assembly 104 is configured to secure to the tube 102 (see FIG. 19) and the head strap 106 is configured to be positioned around the head of a patient. As will be discussed in greater detail herein, the head strap 106 can be a 4-point head strap that secures to the holding assembly 104 at four points to provide enhanced component (e.g., tube) securement and to prevent component displacement (see FIG. 19).

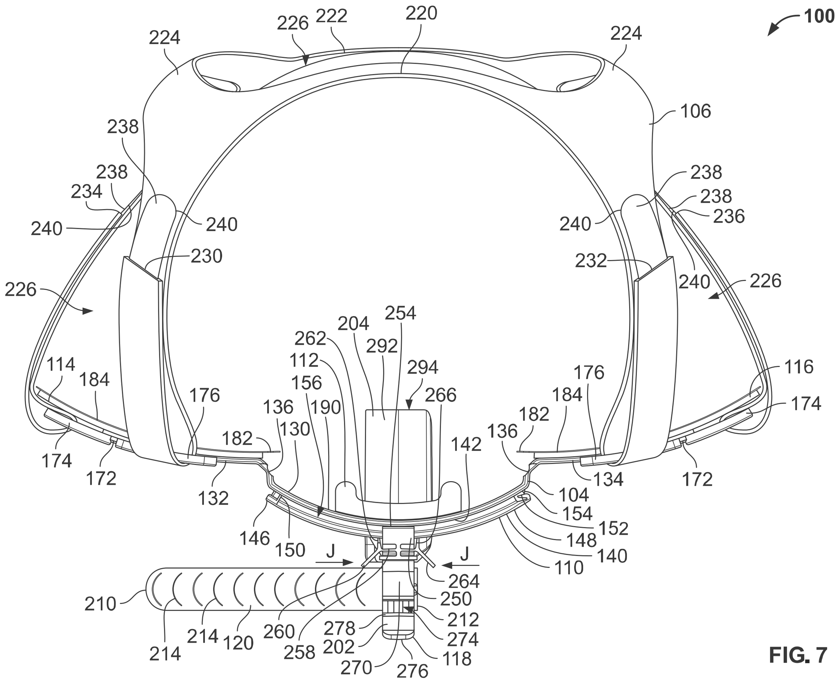

Referring to FIGS. 1 and 2, the holding assembly 104 includes a holding body 110, a face support 112, a first pad 114, a second pad 116, a tube holder 118, and a tube strap 120. The holding body 110 can include a bar 130 that extends from a first cheek bracket 132 to a second cheek bracket 134. The holding body 110 can be transparent or translucent and can be made from a plastic or polymeric material. In some non-limiting examples, the holding body 110 may not be transparent or translucent and may be opaque. As illustrated in FIGS. 1 and 2, the bar 130 is connected to the first cheek bracket 132 and the second cheek bracket 134 at a hinge 136 and the holding body 110 comprises a generally arched shape from above (see FIG. 7). As will be discussed in further details herein, the bar 130, the hinges 136, and the cheek brackets 132, 134 may be bent, slightly deformed, and/or contoured in order to secure to a patient's face. Therefore, the material of the holding body 110 may be flexible and can be bent or formed into different configurations to fit on the patient.

Referring still to FIGS. 1 and 2, the bar 130 is arched and comprises a track 140 extending from a front side 142 of the bar 130. The track 140 can include a plurality of recesses 144 that extend from a first end 146 of the track 140 to a second end 148 of the track 140. A track wall 150 is positioned adjacent to the first end 146 of the track 140 and a track hinge 152 is positioned on the second end 148 of the track 140. A stop 154 extends outwardly from the front side 142 of the bar 130 adjacent to the track hinge 152 (see FIG. 7). As will be discussed in further details herein, the track 140 is configured to secure the tube holder 118 to the holding body 110 between the track wall 150 and the track hinge 152. As illustrated in FIGS. 1 and 2, the track 140 extends outwardly from the bar 130 and forms an upper track cavity 156 and a lower track cavity 158 between the track 140 and the bar 130. The upper track cavity 156 and the lower track cavity 158 extend between the track wall 150 and the track hinge 152.

Referring still to FIGS. 1 and 2, the first and second cheek brackets 132, 134 are positioned on opposite ends of the bar 130 and are mirror images of each other. Each of the first and second cheek brackets 132, 134 include a recessed bracket body 170, a frame 172, a first strap loop 174, and a second strap loop 176. The bracket body 170 of the first cheek bracket 132 is secured with the first pad 114 and the bracket body 170 of the second cheek bracket 134 is secured with the second pad 116. The frame 172 gives additional support to the first and second cheek brackets 132, 134, and the first strap loops 174 and the second strap loops 176 are configured to secure with the head strap 106. As noted herein, the first and second strap loops 174, 176 are secured with the head strap 106 and are positioned to reduce drag on the tube 102 that can cause tube displacement, accidental extubation, and a higher cost of care. Thus, the first and second strap loops 174, 176 are advantageously positioned so that the head strap 106 can properly secure the holding assembly 104 to the patient's face. The first and second strap loops 174, 176 comprise a racetrack shape. However, in some non-limiting examples, the first and second strap loops 174, 176 may comprise any type of shape, *e.g.,* an oval, circular, rectangular, square, triangular, or polygonal shape. Further, in some non-limiting examples, each of the first and second cheek brackets 132, 134 may include more than two strap loops 174, 176. For example, in some non-limiting examples, each of the first and second cheek brackets 132, 134 may include 3, 4, or 5 strap loops 174, 176. Furthermore, in some non-limiting examples, each of the first and second cheek brackets 132, 134 may include just a single strap loop.

Referring still to FIGS. 1 and 2, the first and second pads 114, 116 are mirror images of each other and each include a peel off tab 182 secured to a rear side 184 of the first and second pads 114, 116. The first and second pads 114, 116 are made of a hydrocolloid dressing and each comprise an adhesive (not shown) on the rear side 184. The adhesive is configured to secure the first and second pads 114, 116 to the face or cheek of the patient during use. The first and second pads 114, 116 may be flat or may be contoured to match the shape of a patient's face and/or cheek. As noted herein, the first and second pads 114, 116 are transparent or translucent. Therefore, the patient's face or cheek can be seen or partially seen through the first and second pads 114, 116. This allows medical professionals to see the patient's face or cheek under the first and second pads 114, 116. If the skin of the user gets irritated or red during use, the medical professionals can see this through the first and second pads 114, 116 and adjust the system 100 accordingly or provide a new system 100. Thus, further care can be provided to the patient by way of the transparent or translucent pads 114, 116. In some non-limiting examples, the first and second pads 114, 116 may be opaque. During use, the medical professional can peel off the peel off tabs 182 from the first and second pads 114, 116 to expose the adhesive thereon. The first and second pads 114, 116 can then be applied to the patient's face or cheek to secure the holding assembly 104 to the patient's face.

Referring still to FIGS. 1 and 2, the face support 112 extends from a rear side 190 of the bar 130. The face support 112 may be formed of a foam material. However, in some non-limiting examples, the face support 112 may be formed from a plastic or fabric. The face support 112 comprises a generally U-shape and is configured to be positioned just above the patient's mouth during use. The face support 112 is ideally formed of a soft or gentle material to limit any irritation on the patient.

Referring still to FIGS. 1 and 2, the tube holder 118 includes a tube holder body 202 secured to a bite block 204. In some non-limiting examples, the tube holder 118 may not include the bite block 204. Similar to the holding body 110, the tube holder 118 can be transparent or translucent and formed from a similar or different plastic or polymer material. As illustrated in FIGS. 1 and 2, the tube strap 120 extends outwardly from the tube holder 118 and is secured within a slot 206 in the tube holder 118. The tube strap 120 comprises a first end 210 and a second end 212. The first end 210 of the tube strap 120 is a free end and the second end 212 of the tube strap 120 is secured within the slot 206. Specifically, the second end 212 of the tube strap 120 is thicker than the first end 210 of the tube strap 120, and the second end 212 of the tube strap 120 is press fit within the slot 206 of the tube holder 118. Therefore, the thicker second end 212 of the tube strap 120 cannot slide through the slot 206 of the tube holder 118. The tube strap 120 comprises a plurality of ribs 214 that assist a medical professional or person/user applying the system 100 to the patient with gripping and holding the tube strap 120. In particular, each of the plurality of ribs 214 are shaped in an arc. The arc shape of the plurality of ribs 214 improves the gripping of the tube strap 120 over straight lined ribs because the user or medical professional's fingertips may slide along a straight rib. In contrast, the curve of the arc prevents sliding of the fingertips and allows the fingertip to conform to the curve of the arc, improving the grip at a variety of hand positions and improving ease of use. The radius of each of the plurality of ribs 214 is approximately a diameter of a fingertip. In some non-limiting examples, the plurality of ribs 214 may be small bumps, small circular ribs, and/or other projections. Further, in some non-limiting examples, the plurality of ribs 214 may be perforations into the tube strap 120 instead of ribs protruding out from the tube strap 120. As illustrated in FIG. 2, the tube strap 120 also comprises a tube strap peel off tab 216 positioned over an adhesive (not shown). As will be discussed in further details herein, the tube strap 120 is configured to be wrapped around the tube 102 to secure the tube 102 to the system 100 (see FIG. 19).

Referring still to FIGS. 1 and 2, the head strap 106 is secured with the holding assembly 104 at 4-points, *i.e.,* the first and second strap loops 174, 176 on each of the first and second cheek brackets 132, 134. The head strap 106 includes an upper strap 220 and a lower strap 222. The upper strap 220 is configured to secure with the second strap loops 176 on each of the first and second cheek brackets 132, 134, and the lower strap 222 is configured to secure with the first strap loops 174 on each of the first and second cheek brackets 132, 134. The upper strap 220 is connected to the lower strap 222 by support straps 224. The upper strap 220, the lower strap 222, and the support straps 224 form the head strap 106 and a plurality of openings 226 within the head strap 106. The head strap 106 is configured help better secure the system 100 to the patient's head by preventing the tube 102 from sagging down during use. The upper strap 220 helps keep the tube 102 in place better than single strap configurations and can be very useful for patients with facial hair. As such, the head strap 106 creates more strap connection points to the holding assembly 104 to reduce drag on the tube 102 that can cause tube displacement, accidental extubation, and a higher cost of care. In some non-limiting examples, the system 100 may include a 2-point connection. In other words, in some non-limiting examples, the head strap 106 may secure to just two of the strap loops 174, 176 instead of all four.

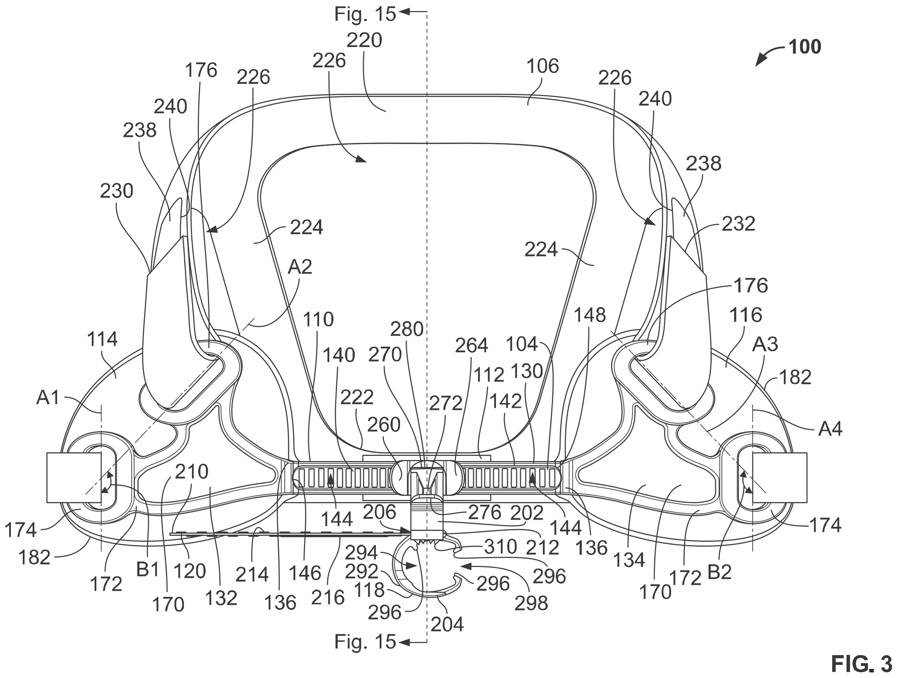

Referring now to FIG. 3, a front of the system 100 is shown. As illustrated in FIG. 3, the first strap loop 174 on the first cheek bracket 132 comprises an axis A1, the second strap loop 176 on the first cheek bracket 132 comprises an axis A2, the second strap loop 176 on the second cheek bracket 134 comprises an axis A3, and the first strap loop 174 on the second cheek bracket 134 comprises an axis A4. The axes A1, A2, A3, A4 extend longitudinally through the center of their respective first and second strap loops 174, 176. As illustrated in FIG. 3, the axis A1 and the axis A4 are substantially perpendicular to the bar 130. In contract, the axis A2 and the axis A3 in FIG. 3 are canted or angled with respect to the bar 130 and the axis A1 and the axis A4.

Referring still to FIG. 3, the first axis A1 and the second axis A2 form an angle B1. In some non-limiting examples, the angle B1 is between about 1° and about 179°, or between about 20° and about 160°, or between about 90° and about 180°, or between about 91° and about 179°, or between about 95° and about 170°, or between about 100° and about 170°, or between about 100° and about 130°, or between about 100° and about 120°, or between about 105° and about 115°, or about 110°, or at least about 91°, or at least about 95°, or at least about 100°, or at least about 105°, or at least about 110°. In some non-limiting examples, the angle B1 is not 90°, *i.e.,* the first axis A1 and the second axis A2 are not perpendicular to one another.

Referring still to FIG. 3, the third axis A3 and the fourth axis A4 form an angle B2. In some non-limiting examples, the angle B2 is between about 1° and about 179°, or between about 20° and about 160°, or between about 90° and about 180°, or between about 91° and about 179°, or between about 95° and about 170°, or between about 100° and about 170°, or between about 100° and about 130°, or between about 100° and about 120°, or between about 105° and about 115°, or about 110°, or at least about 91°, or at least about 95°, or at least about 100°, or at least about 105°, or at least about 110°. In some non-limiting examples, the angle B2 is not 90°, *i.e.,* the third axis A3 and the fourth axis A4 are not perpendicular to one another.

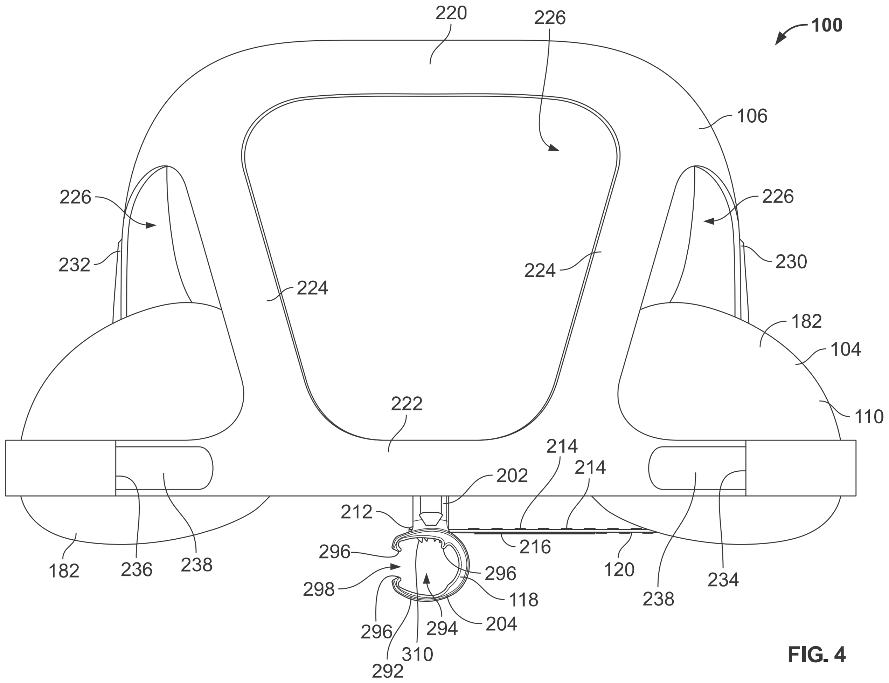

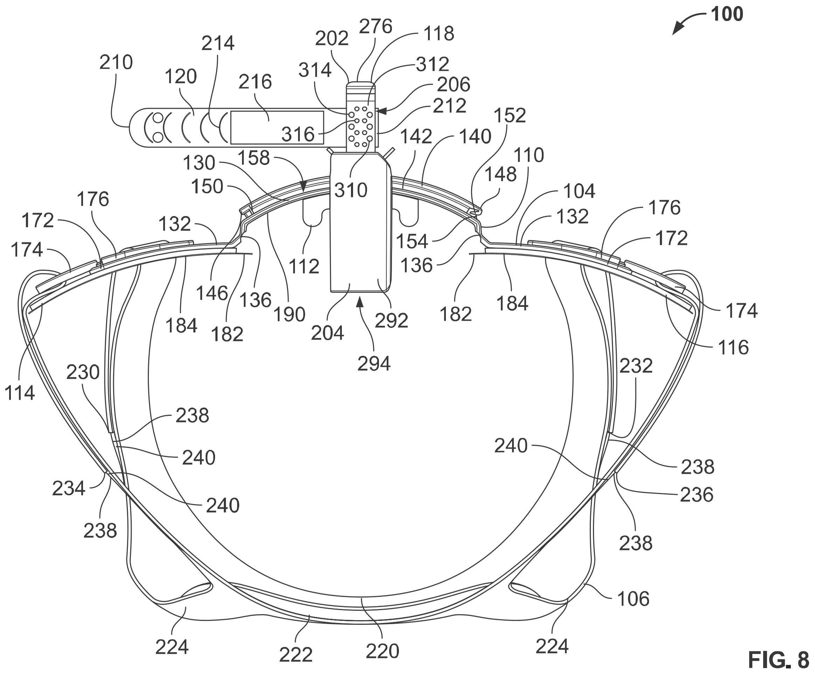

Referring now to FIG. 4, a rear view of the system 100 is shown and the head strap 106 is shown in more detail. As illustrated in FIG. 4, the upper strap 220 comprises a first upper strap end 230 and a second upper strap end 232, and the lower strap 222 comprises a first lower strap end 234 and a second lower strap end 236. Each of the strap ends 230, 232, 234, 236 comprises a strap tab 238 and a securing portion 240 (see FIG. 8) positioned on the strap tab 238. As illustrated in FIGS. 3 and 4, the securing portions 240 are configured to secure the strap ends 230, 232, 234, 236 (and the strap tabs 238) to their respective strap 220, 222. In other words, during use, the first upper strap end 230 of the upper strap 220 is configured to extend through the second strap loop 176 of the first cheek bracket 132 and secure back with a portion of the upper strap 220. Similarly, the second upper strap end 232 of the upper strap 220 is configured to extend through the second strap loop 176 of the second cheek bracket 134 and secure back with a portion of the upper strap 220. The first lower strap end 234 of the lower strap 222 is configured to extend through the first strap loop 174 of the first cheek bracket 132 and secure back with a portion of the lower strap 222. The second lower strap end 236 of the lower strap 222 is configured to extend through the first strap loop 174 of the second cheek bracket 134 and secure back with a portion of the lower strap 222. Therefore, the securing portions 240 on each of the upper strap ends 230, 232 are configured to secure the upper strap ends 230, 232 back to the upper strap 220, and the securing portions 240 on each of the lower strap ends 234, 236 are configured to secure the lower strap ends 234, 236 back to the lower strap 222. As such, the user or medical professional can adjust the head strap 106 at the 4 points of contact with the holding assembly by way of the upper and lower strap ends 230, 232, 234, 236 to fit the system 100 to the patient's head and secure the system 100 to the patient's head. In some non-limiting examples, the securing portions 240 may be Velcro^{®} or a similar securing element that is secured to a portion of the upper strap 220 and the lower strap 222. Therefore, in such an embodiment, the head strap 106 or portions of the head strap 106 may be made from a material that secures with the securing portions 240 on each of the strap tabs 238. Further, in some non-limiting examples, the securing portions 240 may be a buckle (cinch locks, ladder locks, and/or tri-glides), an adhesive, and a slide release buckle that secures the upper and lower strap ends 230, 232, 234, 236 to the head strap 106.

As noted herein, the upper and lower strap ends 230, 232, 234, 236 are shown secured with an outside/outer end of the head strap 106 in FIGS. 1-8. However, in some non-limiting examples, the head strap 106 may be flipped such that the upper and lower strap ends 230, 232, 234, 236 are secured with an inside/inner end of the head strap 106. Further, it is contemplated that the outside/outer end of the head strap 106 and/or the inside/inner end of the head strap # may comprise of a material that secures with the securing portions 240, *e.g.,* Velcro^{®} connection.

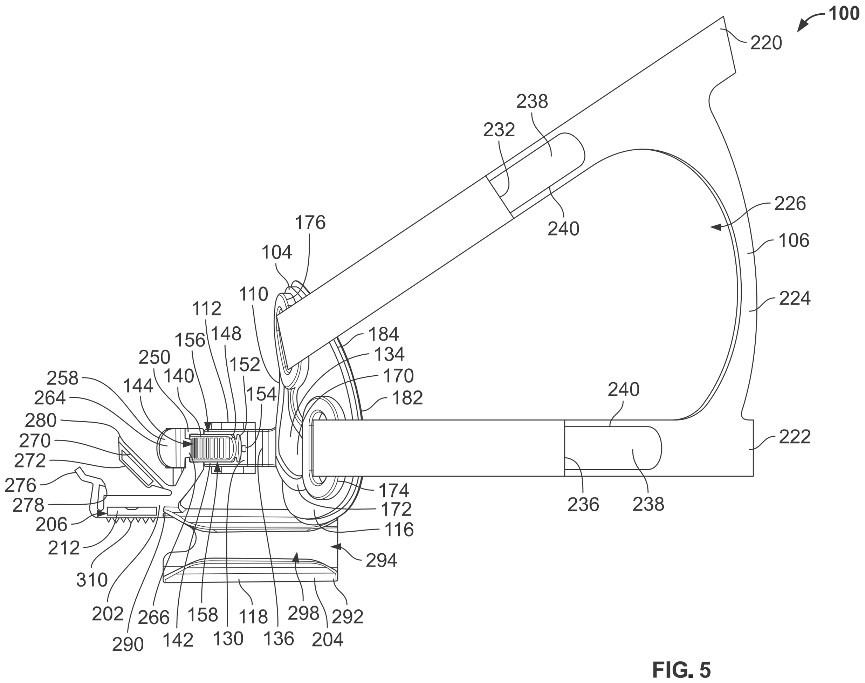

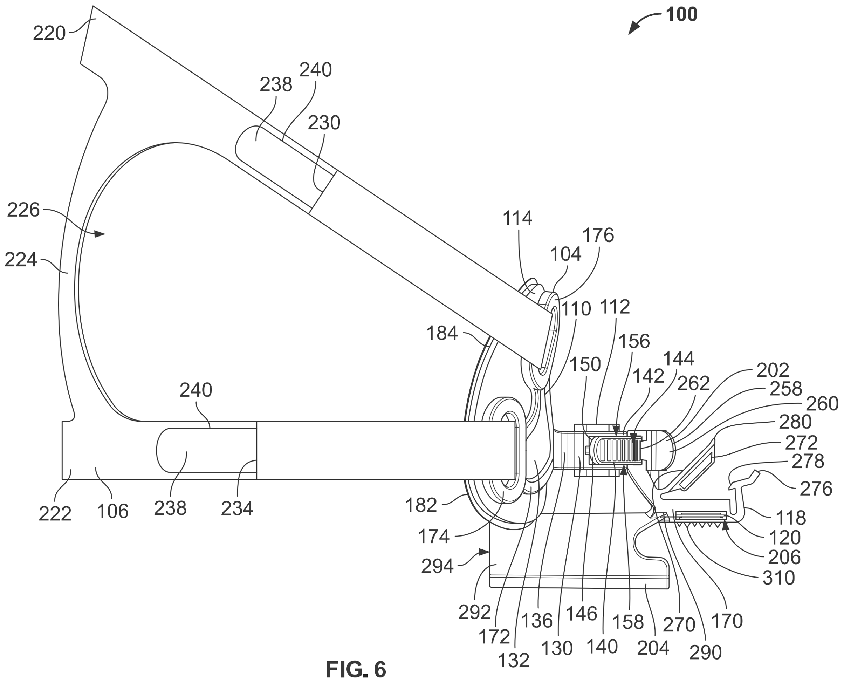

Referring now to FIGS. 5 and 6, a right side view and a left side view of the system is shown, respectively. As illustrated in FIGS. 5 and 6, the contour of the first and second cheek brackets 132, 134 and the first and second pads 114, 116 can be seen in more detail. As noted herein, the first strap loop 174 and the second strap loop 176 on the second cheek bracket 134 are offset with respect to each other in all three dimensions (see FIG. 5), and the first strap loop 174 and the second strap loop 176 on the first cheek bracket 132 are offset with respect to teach other in all three dimensions (see FIG. 5).

Referring now to FIGS. 7 and 8, a top view and a bottom view of the system 100 is shown in more detail, respectively. As illustrated in FIGS. 7 and 8, the holding assembly 104 comprises a wing shaped and is generally convex when referencing it from the front. As discussed above, the shape of the holding assembly 104 is arched or wing shaped so that it can fit on the contours of the patient's face. As also discussed above, the holding assembly 104 is bendable such that it can secure to the patient's face.

Referring to FIGS. 1-8, the method of securing the system 100 to the patient will be described below. Specifically, first the head strap 106 of the system 100 is placed over the patient's head. Once the head strap 106 is placed over a patient's head, the user or medical professional can adjust the head strap 106 and peel off the peel off tabs 182 of the first and second pads 114, 116 to apply the pads 114, 116 to the patient's face. The holding assembly 104 can be bent so that the adhesive on the first and second pads 114, 116 can come into contact with the cheeks or face of the patient. The adhesive on the first and second pads 114, 116 will secure the pads 114, 116 to the cheeks or face of the patient. The face support 112 should be positioned between the patient's nose and mouth once the system is secured to the patient. After the first and second pads 114, 116 are attached to the cheek or face of the patient, the head strap 106 can be further adjusted appropriately to have the best fit for the patient. The upper strap 220 and the lower strap 222 can be altered by undoing the securing portion 240 on each of the strap tabs 238 and tightening or loosening the upper strap 220 and/or the lower strap 222 until the desired position is achieved. The securing portion 240 on each of the strap tabs 238 can then be secured with their respective upper and lower strap 220, 222 to secure the straps 220, 222 in place. In some non-limiting embodiments, the holding assembly 104 may be secured to the patient's face without the head strap 106 attached with the holding assembly 104. Once the holding assembly 104 is secured to the patient's face by way of the first and second pads 114, 116, the head strap 106 then may be secured with the holding assembly 104 and to the patient.

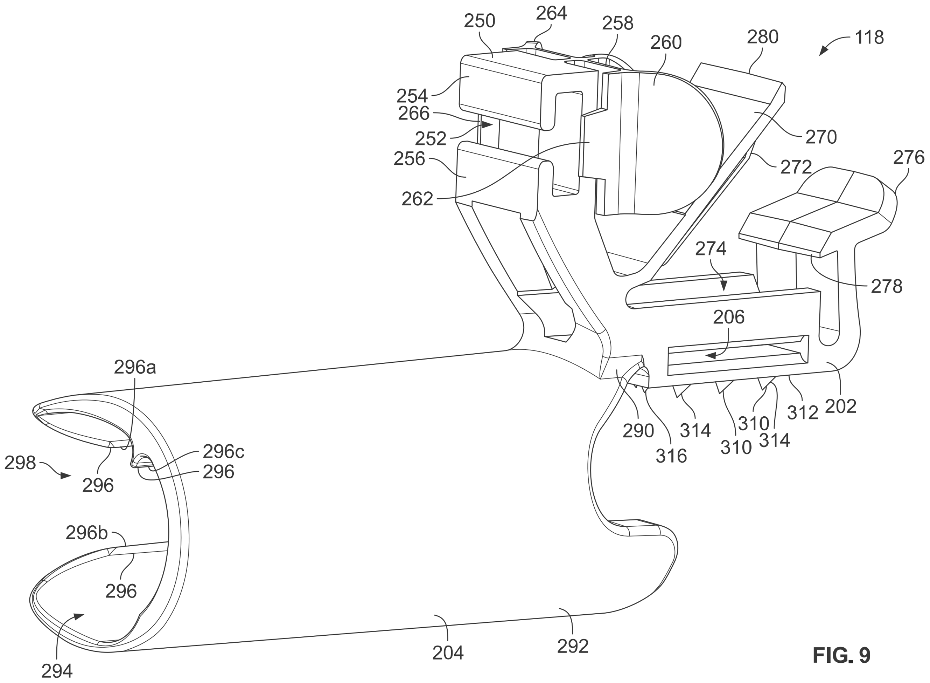

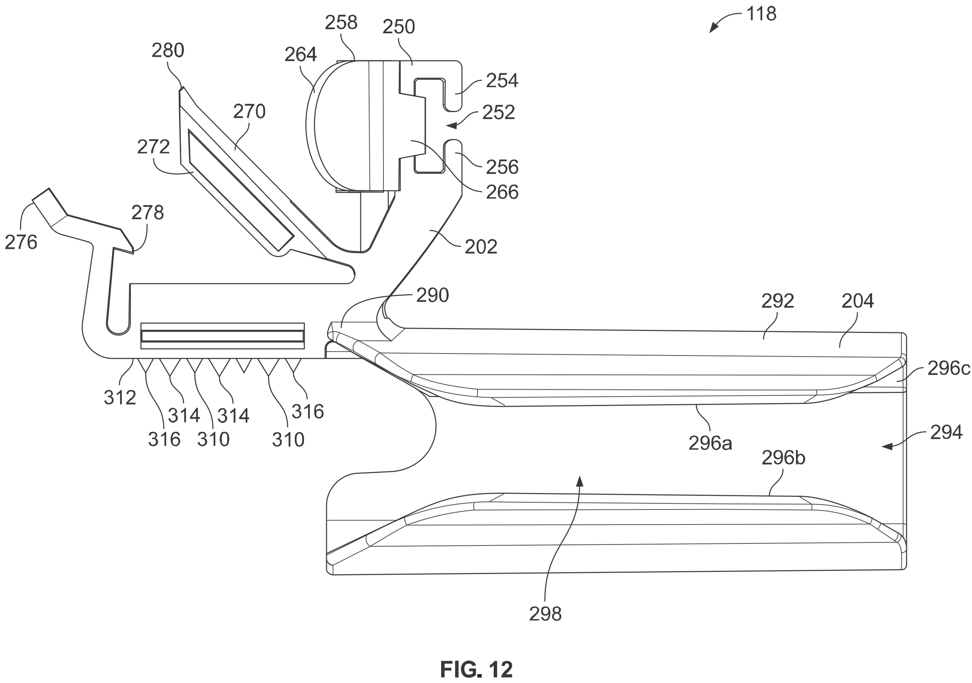

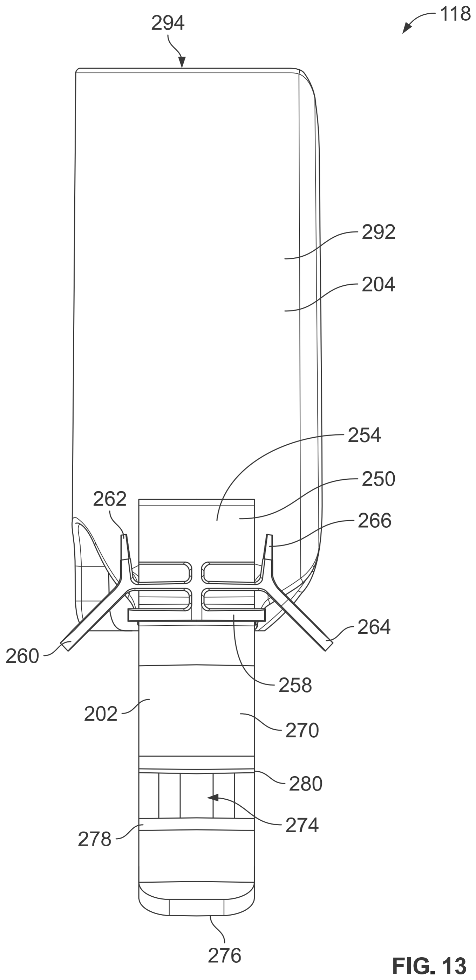

Referring now to FIGS. 9-14, the tube holder 118 is shown in more detail. As illustrated in FIGS. 9, the tube holder body 202 comprises a shuttle 250 with a shuttle chamber 252 extending therethrough. The shuttle 250 comprises a first arm 254 and a second arm 256 that partially surround and define the shuttle chamber 252. As will be discussed in greater details herein, shuttle 250 is configured to wrap partially around the track 140 of bar 130 to secure the tube holder 118 to the holding body 110. As illustrated in FIGS. 9 and 12, the tube holder body 202 also comprises a latch 258 positioned in front of the shuttle 250. The latch 258 comprises a first wing 260 connected with a first prong 262 and a second wing 264 connected with a second prong 266. The first and second prongs 262, 266 extend adjacent to sides of the shuttle 250. In other words, a portion of the shuttle 250 is positioned between the first and second prongs 262, 266. As will be discussed in greater detail herein, the first and second prongs 262, 266 are configured to extend into the plurality of recesses 144 within the track 140 to secure the tube holder 118 in place.

Referring still to FIGS. 9 and 12, the tube holder body 202 further comprises a securing flange 270 that extends therefrom. The securing flange 270 comprises a securing rib 272 and is positioned over a channel 274. The securing flange 270 is configured to interact with a nose 276 of the tube holder body 202. Specifically, the nose 276 of the tube holder body 202 comprise a protrusion 278 that is configured to secure a free end 280 of the securing flange 270 in a down position (see FIG. 19). FIGS. 9 and 12 illustrate the securing flange 270 in an upright or open position. During use and when in the down position (see FIG. 19), the securing flange 270 is configured to secure the tube strap 120 within the channel 274 and between the tube holder body 202 and the securing rib 272.

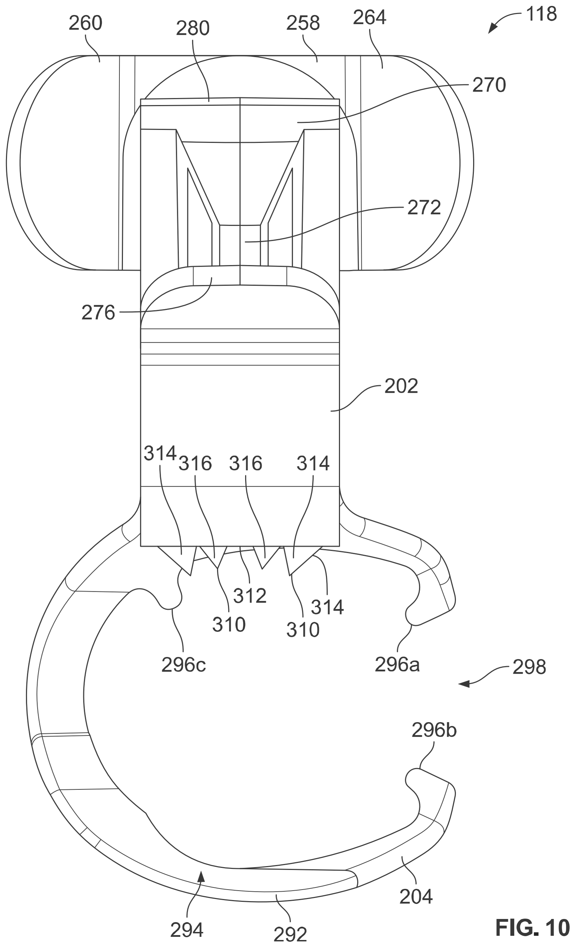

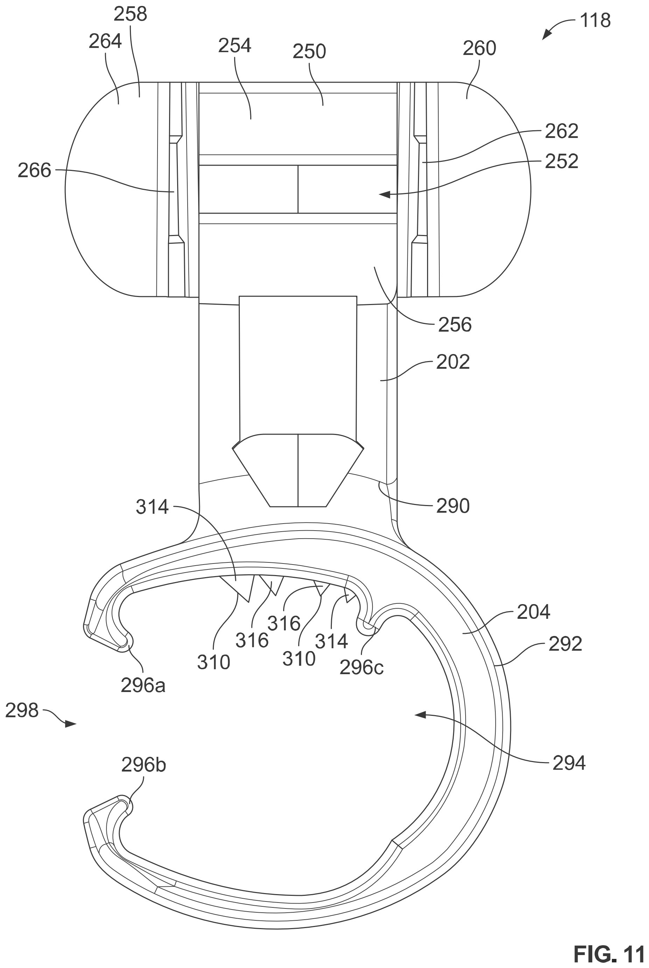

Referring to FIGS. 10 and 12, the bite block 204 extends from a lower end 290 of the tube holder body 202 and in a rearward direction, i.e., away from the nose 276. The bite block 204 is tubular and has a sidewall 292 that defines a bite block channel 294. The bite block channel 294 extends through the bite block 204. As illustrated in FIGS. 10 and 11, the sidewall 292 of the bite block 204 only partially surrounds the bite block channel 294 and the bite block 204 comprises a plurality of fangs 296. As illustrated in FIG. 11, a first fang 296a and a second fang 296b of the plurality of fangs 296 extend from ends of the bite block 204 and into the bite block channel 294. The first and second fangs 296a, 296b define a sidewall opening 298 into the bite block channel 294. The bite block 204 further comprises a third fang 296c extending into the bite block channel 294 near an upper end of the bite block 204. The third fang 296c is spaced from the first and second fangs 296a, 296b and extends along the entire bite block sidewall 292. During use, the bite block 204 is configured to extend between the teeth within a patient's mouth and the tube 102 is configured to extend through the bite block channel 294. In some non-limiting examples, the bite block 204 may be molded with the tube holder 118 to define a single integral piece. Further, in some non-limiting examples, the bite block 204 may be a separate element secured or attached with the tube holder 118. As noted herein, the plurality of fangs 296 are configured to secure the tube 102 within the bite block channel 294.

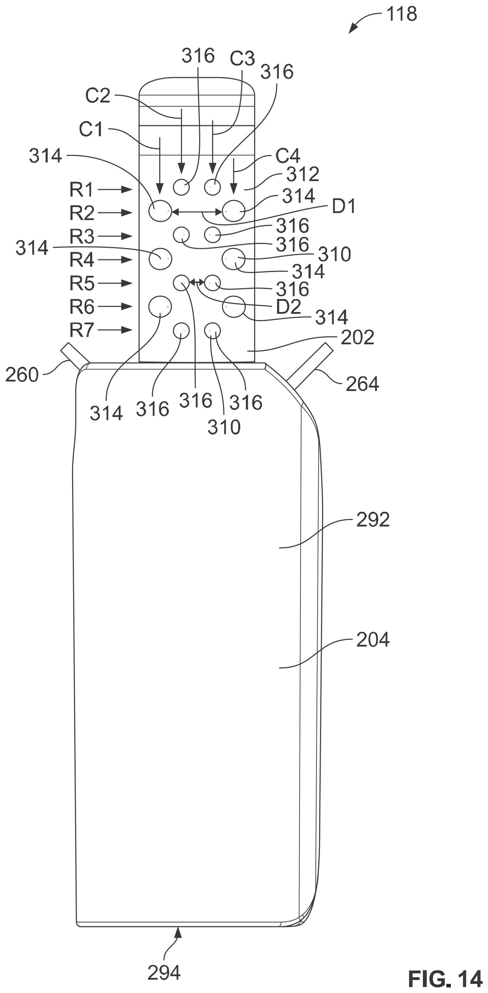

Referring to FIGS. 10, 12, and 14, a plurality of teeth 310 extend from a bottom surface 312 of the tube holder body 202. The plurality of teeth 310 define a conical shape, and the plurality of teeth 310 are positioned in four columns C1, C2, C3, C4 and seven rows R1, R2, R3, R4, R5, R6, R7 (see FIG. 14). The plurality of teeth 310 include a plurality of first teeth 314 and a plurality of second teeth 316. The plurality of first teeth 314 are larger than the plurality of second teeth 316. Specifically, as illustrated in FIGS. 10 and 12, the plurality of first teeth 314 extend farther away from the bottom surface 312 of the tube holder body 202 than the plurality of second teeth 316, and the plurality of first teeth 314 comprise a greater diameter at their base, *i.e.,* section secured with the bottom surface 312 of the tube holder body 202, than the plurality of second teeth 316. As illustrated in FIG. 14, the plurality of first teeth 314 are positioned on outer portions of the bottom surface 312 of the tube holder body 202 (columns C1 and C4) and the plurality of second teeth are positioned between the plurality of first teeth 314 (columns C2 and C3). The plurality of teeth 310 are positioned in pairs along the rows R1, R2, R3, R4, R5, R6, R7. In other words, two of the plurality of second teeth 316 along the row R1 form a pair, two of the plurality of first teeth 314 along the row R2 form a pair, and so on. Each pair of the plurality of first teeth 314 are positioned a distance D1 between each other and each pair of the plurality of second teeth 316 are positioned a distance D2 between each other. As illustrated in FIG. 14, D1 is greater than D2. In some non-limiting examples, the distance D1 is between about 1.0 mm and about 10.0 mm, or between about 2.0 mm and about 6.0 mm, or between about 3.0 mm and about 5.0 mm, or between about 4.0 mm and about 5.0 mm, or about 4.0 mm, or at least about 3.0 mm, or at least about 4.0 mm. In some non-limiting examples, the distance D2 is between about 0.5 mm and about 4 mm, or between about 1.0 mm and about 2.0 mm, or about 1.0 mm, or about 1.2 mm, or at least about 1.0 mm.

Referring to FIG. 14, each pair of the plurality of teeth 310 are offset from each other when extending along a forward-rear direction (see direction of arrows for columns C1, C2, C3, C4). In other words, the pair of the plurality of teeth 310 along the row R1, which includes two of the plurality of second teeth 316 (one in column C2 and one in column C3), is spaced along the forward-rear direction from the pair of the plurality of teeth 310 along the row R2, which includes two of the plurality of first teeth 314 (one in column C1 and one in column C4). As illustrated in FIG. 14, all of the pairs of the plurality of teeth 310 are offset or spaced from other pairs along the forward-rear direction.

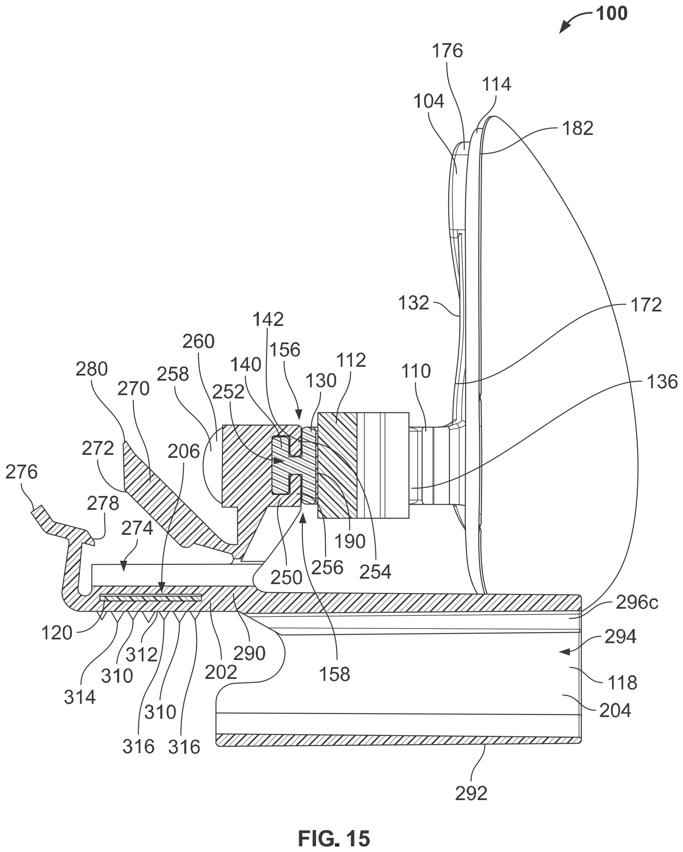

Referring now to FIG. 15, a cross sectional view of the holding assembly 104 is illustrated. As illustrated in FIG. 15, the track 140 is positioned within the shuttle chamber 252 of the shuttle 250 and the tube holder 118 is secured with the holding body 110. The first arm 254 of the shuttle 250 is positioned within the upper track cavity 156 and the second arm 256 of the shuttle 250 is positioned within the lower track cavity 158. As illustrated in FIGS. 1, 5, 6, 7, and 15, the first prong 262 of the latch 258 is positioned within one of the plurality of recesses 144 on the track 140 and the second prong 266 of the latch 258 is positioned within one of the plurality of recesses 144 on the track 140. Therefore, the combination of the track 140 being positioned within the shuttle chamber 252 and the engagement of the prongs 262, 266 with the plurality of recesses 144 causes the tube holder 118 to be secured with the holding body 110. During use, the first and second wings 260, 264 of the latch 258 can act as hinges such that movement of the wings 260, 264 in the direction of arrow J (se FIG. 7) causes the first and second prongs 262, 266 to move outwardly and out from the plurality of recesses 144. Once the prongs 262, 266 are not secured with the plurality of recesses 144, the tube holder 118 can slide along the track 140 between the track wall 150 and the track hinge 152. This allows for movement and adjustment of the tube holder 118 relative to the holding body 110. As noted herein, the tube holder 118 can slide onto the track 140 by way of the track hinge 152 folding in (see FIG. 7). Once placed on the track 140, the track hinge 152 blocks the shuttle 250 of the tube holder 118 from sliding off the track 140 due to the stop 154 blocking the track hinge 152 from folding out (see FIG. 7).

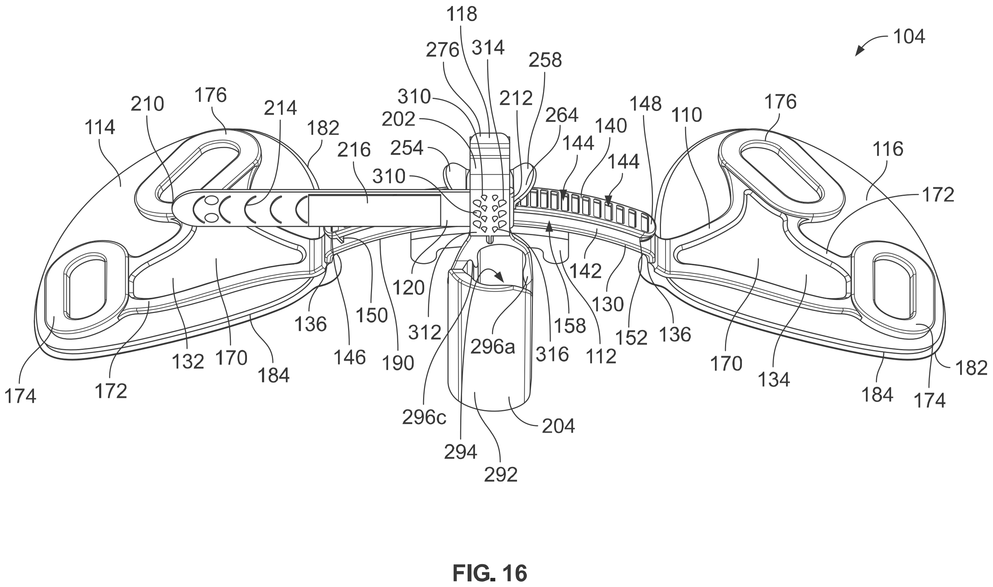

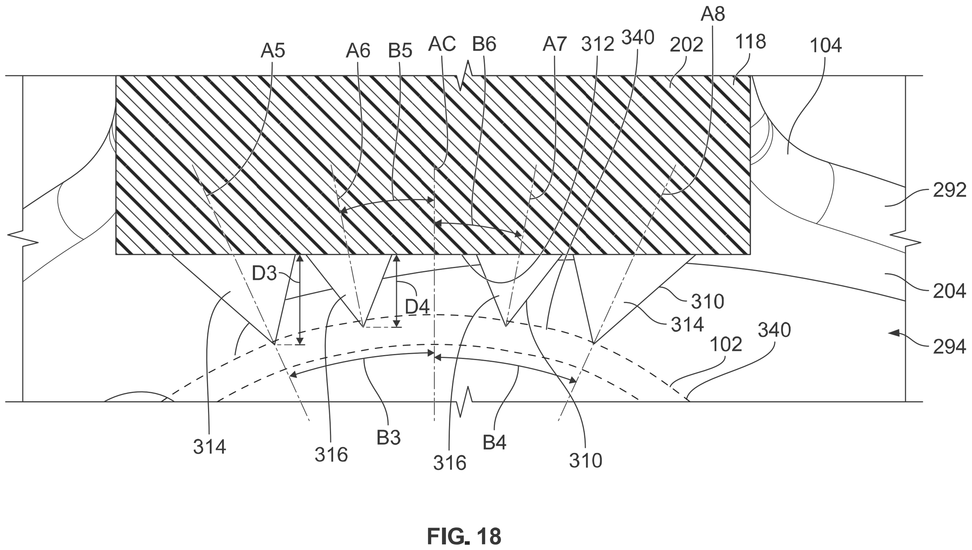

Referring now to FIG. 16, the plurality of teeth 310 are shown in more detail. As will be discussed in greater detail herein, the plurality of teeth 310 are angled differently depending on the column C1, C2, C3, C4 they are in. The plurality of teeth 310 are angled such that the axis of each of the plurality of teeth are configured to be substantially perpendicular to the tube 102 when the tube 102 is secured with the system 100 (see FIG. 18). Thus, all of the plurality of teeth 310 come into contact with the tube 102 when the tube 102 is attached to the system 100. This helps to improve the grip between the system 100 and the tube 102.

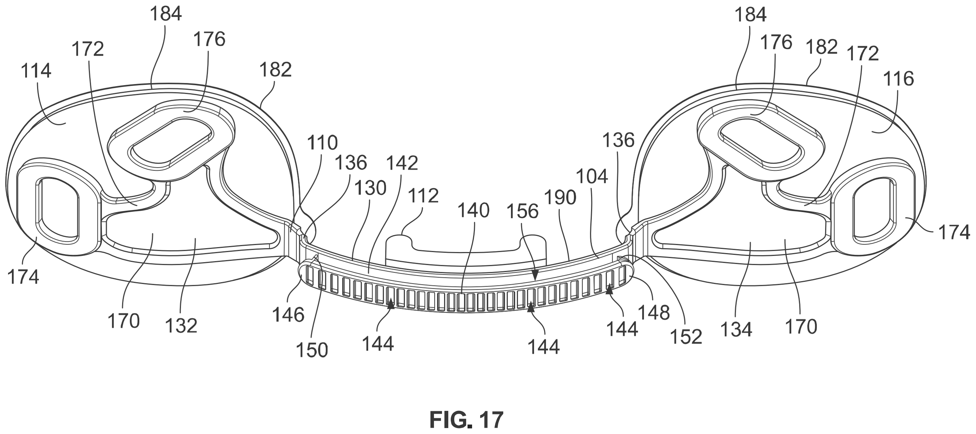

Referring now to FIG. 17, a top isometric view of the holding body 110, the face support 112, the first pad 114, and the second pad 116 is illustrated. The track 140 with the plurality of recesses 144 are illustrated in more detail. In some non-limiting examples, the track 140 may include more or less recesses 144 than illustrated. Further, in some non-limiting examples, the face support 112 may be integral with the holding body 110 and not a separate component.

Referring now to FIG. 18 an enlarged, partial cross-sectional view of the tube holder 118 is illustrated with the tube 102 shown in dash-dash-dash lines. As discussed above, each column C1, C2, C3, C4 of the plurality of teeth 310 is angled differently than another column C1, C2, C3, C4. Specifically, each of the plurality of first teeth 310 in the column C1 comprises an axis A5, each of the plurality of second teeth 316 in the column C2 comprises an axis A6, each of the plurality of second teeth 316 in the column C3 comprises an axis A7, and each of the plurality of first teeth 314 in the column C4 comprises an axis A8. As illustrated in FIG. 18, each of the axes A5, A6, A7, A8 are angled from one another within each respective row R1, R2, R3, R4, R5, R6, R7, *i.e.,* each of the axes A5, A6, A7, A8 are not parallel with another axes A5, A6, A7, A8 along the same row R1, R2, R3, R4, R5, R6, R7 of the plurality of teeth 310. As noted herein, each of the axes A5, A6, A7, A8 extend through a center of a base and a tip of their respective tooth 310 (see FIG. 18).

Referring still to FIG. 18, during use, the plurality of teeth 310 are configured to come into contact with the tube 102. As discussed above, the plurality of teeth 310 are angled so that the axes A5, A6, A7, A8 are substantially perpendicular to an outer surface 340 of the tube 102 and are positioned so that all of the plurality of teeth 310 come into contact with the tube 102 when the tube 102 is secured to the system 100. This contact between the tube 102 and the plurality of teeth 310 allows for improved griping between the system 100 and the tube 102.

Referring still to FIG. 18, the axis A5 and the axis A8 form an angle B3 and an angle B4 with a longitudinal center plane AC (perpendicular to the bottom surface 312 of the tube holder 118 and extending through the center of the bottom surface 312 of the tube holder 118 as illustrated in FIG. 18), respectively, and the axis A6 and the axis A7 form an angle B5 and an angle B6 with the longitudinal center plane AC, respectively. In some non-limiting examples, each of the angle B3 and the angle B4 is between about 0° and about 60°, or between about 5° and about 30°, or between about 10° and about 25°, or between about 15° and about 20°, or about 18°, or at least about 10°, or at least about 15°, or at least about 18°. In some non-limiting examples, each of the angle B5 and the angle B6 is between about 1° and about 60°, or between about 3° and about 30°, or between about 5° and about 15°, or between about 5° and about 10°, or about 8°, or at least about 5°, or at least about 8°. The configuration of the plurality of teeth 310 allow for each of the plurality of teeth 310 to penetrate the tube 102 with approximately the same force and depth. Further, as discussed above, angling or tilting the centerline/axis of the plurality of teeth 310 allows the plurality of teeth 310 to be substantially perpendicular to the tube 102. Thus, the plurality of teeth 310 provide maximum resistance in both a rotational and lateral motion.

As noted herein, the axes A5, A6, A7, A8 correspond to the plurality of teeth 310 positioned within the first two rows R1, R2 (see FIG. 14). However, as illustrated in FIGS. 10, 11, 12, 14, and 18, the plurality of teeth 310 within each column C1, C2, C3, C4 have axes that are parallel. In other words, the plurality of teeth 310 within each column C1, C2, C3, C4 are identical and are just spaced from each other along the column C1, C2, C3, C4. For example, the first tooth 314 in column C1 and row R2 is identical to the first tooth 314 in column C1 and row R4 and is identical to the first tooth 314 in column C1 and row R6. In some non-limiting examples, the plurality of teeth 310 within each column C1, C2, C3, C4 may not be the same or identical throughout each column C1, C2, C3, C4, *i.e.,* each of the plurality of teeth 310 within a column are different than each other.

Referring still to FIG. 18, the axes A5, A6, A7, A8 of each of the plurality of teeth 310 are not parallel with the longitudinal center plane AC. As illustrated in FIG. 18, each of the axes A5, A6, A7, A8 of the plurality of teeth 310 converge toward the longitudinal center plane AC below the bottom surface 312. In fact, each of the axes A5, A6, A7, A8 within a single row R1, R2, R3, R4, R5, R6, R7 converge toward each other below the bottom surface 312.

Referring still to FIG. 18, the plurality of first teeth 314 comprise a distance D3 between the bottom surface 312 of the tube holder body 202 and a tip of the plurality of first teeth 314. The plurality of second teeth 316 comprise a distance D4 between the bottom surface 312 of the tube holder body 202 and a tip of the plurality of second teeth 316. As discussed above, the distance D3 is larger than the distance D4. In some non-limiting examples, the distance D3 is between about 0.5 mm and about 5 mm, or between about 0.8 mm and about 3.0 mm, or between about 1.0 mm and about 1.5 mm, or about 1.3 mm, or about 1.4 mm, or at least about 0.5 mm, or at least about 1.0 mm, or at least about 1.3 mm. In some non-limiting examples, the distance D4 is between about 0.1 mm and about 5 mm, or between about 0.5 mm and about 3 mm, or between about 0.8 mm and about 1.3 mm, or about 1.0 mm, or about 1.1 mm, or at least about 0.5 mm, or at least about 1.0 mm. In some non-limiting examples, the distance D3 is between about 10% and about 50% larger than the distance D4, or the distance D3 is about 28% larger than the distance D4.

Referring now to FIG. 19, the system 100 is illustrated attached to the tube 102. As illustrated in FIG. 19, the tube strap 120 is positioned around the tube 102 and secured within the channel 274 and under the securing flange 270. Referring to FIGS. 1, 9, 12, 15, and 19, a method of attaching the tube 102 to the tube holder 118 of the system 100 is describe below. Specifically, once the system 100 is secured to the patient, the user or medical professional can place the tube 102 into the patient and through the bite block channel 294. The tube 102 can be positioned within the bite block channel 294 through the sidewall opening 298 of the bite block 204. Once the tube 102 is placed within the bite block channel 294, the tube 102 will be aligned with the tube holder 118 and the plurality of teeth 310. Then, the user or medical professional can wrap the tube strap 120 around the tube 102 to secure the tube 102 to the tube holder 118. Before wrapping the tube strap 120 around the tube 102, the user or medical professional can remove the tube strap peel off tab 216 to expose the adhesive. The adhesive can then be secured to the tube 102 as the tube strap 120 is wrapped around the tube 102. The adhesive helps secure the tube strap 120 to the endotracheal tube 102. Once wrapped around the tube 102, the tube strap 120 should be positioned above the channel 274 and under the securing flange 270. Once the tube strap 120 is positioned under the securing flange 270, the user or medical professional can clamp the securing flange 270 down to the down position. In the down position, the protrusion 278 will jam a portion of the tube strap 120 into the channel 274 and lock the tube strap 120 in place. To secure the securing flange 270 down to the down position, the user or medical professional can urge the nose 276 of the tube holder 118 away from the tube strap 120 to allow the protrusion 278 to move out of the way and to allow the securing flange 270 to pivot to the down position. Once the securing flange 270 is in the down position, the user or medical professional can let go of the nose 276 of the tube holder 118 and the protrusion 278 will lock the free end 280 of the securing flange 270 in the down position.

Referring to FIGS. 18 and 19, once the tube strap 120 has secured the tube 102 to the tube holder 118, the tube 102 will be in contact and secured with the plurality of teeth 310. As discussed above, the angle of the plurality of teeth 310 allow for better securement of the tube 102 to the tube holder 118. As noted herein, if the system 100 does not have the bite block 2004, the user or medical professional can then start directly with wrapping the tube strap 120 around the tube 102 once the system 100 is placed on the patient and the tube 102 is positioned within the user, *i.e.,* the steps of placing the tube 102 into the bite block 204 can be removed. In some non-limiting examples, the tube 102 may be placed within the patient before the system 100 is secured to the patient.

Once the tube 102 is secured with the tube holder 118, the tube holder 118 can be adjusted by moving the tube holder 118 along the track 140 of the holding body 110. Specifically, as discussed above, the first and second prongs 262, 266 of the latch 258 can be moved out from the recesses 144 on the track 140 by movement of the wings 260, 264. Once the wings 260, 264 are clamped in the direction of arrow J (see FIG. 7), the tube holder 118 can slide along the track 140 until the appropriate position is achieved. Since the tube 102 is secured with the tube holder 118, the tube 102 can slide with the tube holder 118 along the track 140. Once the appropriate position is achieved, the user or medical professional can let go of the wings 260, 264 to allow the first and second prongs 262, 266 to snap back into the recesses 144 to secure the tube holder 118 in a static position on the track 140.

As noted herein, the system 100 provides enhanced endotracheal tube securement while preventing endotracheal tube displacement. The system 100 also helps to minimize pressure on the upper lips of a patient and helps decrease the risk of skin irritation. Thus, due to the four point securement with the head strap 106 and holding assembly 104, the system 100 allows for better securement, better comfort, and a reduced risk of unplanned extubation, pressure ulcers, and skin damage. The system 100 also will not occlude the tube 102. Therefore, the system 100 improves retention of the tube 102.

The present disclosure has described one or more preferred non-limiting examples, and it should be appreciated that many equivalents, alternatives, variations, and modifications, aside from those expressly stated, are possible and within the scope of the invention.

It is to be understood that the disclosure is not limited in its application to the details of construction and the arrangement of components set forth in the accompanying description or illustrated in the accompanying drawings. The disclosure is capable of other non-limiting examples and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Unless specified or limited otherwise, the terms "mounted," "connected," "supported," and "coupled" and variations thereof are used broadly and encompass both direct and indirect mountings, connections, supports, and couplings. Further, "connected" and "coupled" are not restricted to physical or mechanical connections or couplings.

As used herein, unless otherwise limited or defined, discussion of particular directions is provided by example only, with regard to particular non-limiting examples or relevant illustrations. For example, discussion of "top," "front," or "back" features is generally intended as a description only of the orientation of such features relative to a reference frame of a particular example or illustration. Correspondingly, for example, a "top" feature may sometimes be disposed below a "bottom" feature (and so on), in some arrangements or non-limiting examples. Further, references to particular rotational or other movements (e.g., counterclockwise rotation) is generally intended as a description only of movement relative a reference frame of a particular example of illustration.

Certain operations of methods according to the disclosure, or of systems executing those methods, may be represented schematically in the figures, or otherwise discussed herein. Unless otherwise specified or limited, representation in the figures of particular operations in particular spatial order may not necessarily require those operations to be executed in a particular sequence corresponding to the particular spatial order. Correspondingly, certain operations represented in the figures, or otherwise disclosed herein, can be executed in different orders than are expressly illustrated or described, as appropriate for particular non-limiting examples of the disclosure. Further, in some non-limiting examples, certain operations can be executed in parallel, including by dedicated parallel processing devices, or separate computing devices configured to interoperate as part of a large system.

In some implementations, devices or systems disclosed herein can be utilized or installed using methods embodying aspects of the disclosure. Correspondingly, description herein of particular features, capabilities, or intended purposes of a device or system is generally intended to inherently include disclosure of a method of using such features for the intended purposes, a method of implementing such capabilities, and a method of installing disclosed (or otherwise known) components to support these purposes or capabilities. Similarly, unless otherwise indicated or limited, discussion herein of any method of manufacturing or using a particular device or system, including installing the device or system, is intended to inherently include disclosure, as non-limiting examples of the disclosure, of the utilized features and implemented capabilities of such device or system.

As used herein, unless otherwise defined or limited, ordinal numbers are used herein for convenience of reference based generally on the order in which particular components are presented for the relevant part of the disclosure. In this regard, for example, designations such as "first," "second," etc., generally indicate only the order in which the relevant component is introduced for discussion and generally do not indicate or require a particular spatial arrangement, functional or structural primacy or order.

As used herein, unless otherwise defined or limited, directional terms are used for convenience of reference for discussion of particular figures or examples. For example, references to downward (or other) directions or top (or other) positions may be used to discuss aspects of a particular example or figure, but do not necessarily require similar orientation or geometry in all installations or configurations.

This discussion is presented to enable a person skilled in the art to make and use non-limiting examples of the disclosure. Various modifications to the illustrated examples will be readily apparent to those skilled in the art, and the generic principles herein can be applied to other examples and applications without departing from the principles disclosed herein. Thus, non-limiting examples of the disclosure are not intended to be limited to non-limiting examples shown but are to be accorded the widest scope consistent with the principles and features disclosed herein and the claims below. The accompanying detailed description is to be read with reference to the figures, in which like elements in different figures have like reference numerals. The figures, which are not necessarily to scale, depict selected examples and are not intended to limit the scope of the disclosure. Skilled artisans will recognize the examples provided herein have many useful alternatives and fall within the scope of the disclosure.

Also as used herein, unless otherwise limited or defined, "or" indicates a non-exclusive list of components or operations that can be present in any variety of combinations, rather than an exclusive list of components that can be present only as alternatives to each other. For example, a list of "A, B, or C" indicates options of: A; B; C; A and B; A and C; B and C; and A, B, and C. Correspondingly, the term "or" as used herein is intended to indicate exclusive alternatives only when preceded by terms of exclusivity, such as "either," "one of," "only one of," or "exactly one of." Further, a list preceded by "one or more" (and variations thereon) and including "or" to separate listed elements indicates options of one or more of any or all of the listed elements. For example, the phrases "one or more of A, B, or C" and "at least one of A, B, or C" indicate options of: one or more A; one or more B; one or more C; one or more A and one or more B; one or more B and one or more C; one or more A and one or more C; and one or more of each of A, B, and C. Similarly, a list preceded by "a plurality of" (and variations thereon) and including "or" to separate listed elements indicates options of multiple instances of any or all of the listed elements. For example, the phrases "a plurality of A, B, or C" and "two or more of A, B, or C" indicate options of: A and B; B and C; A and C; and A, B, and C. In general, the term "or" as used herein only indicates exclusive alternatives (e.g. "one or the other but not both") when preceded by terms of exclusivity, such as "either," "one of," "only one of," or "exactly one of."

Also as used herein, unless otherwise specified or limited, the terms "about" and "approximately," as used herein with respect to a reference value, refer to variations from the reference value of ± 5% or less (e.g., ± 3%, ± 1%, etc.), inclusive of the endpoints of the range. Similarly, the term "substantially equal" (and the like) as used herein with respect to a reference value refers to variations from the reference value of less than ± 30% (e.g., ± 20%, ± 10%, ± 5%) inclusive. Where specified, "substantially" can indicate in particular a variation in one numerical direction relative to a reference value. For example, "substantially less" than a reference value (and the like) indicates a value that is reduced from the reference value by 30% or more, and "substantially more" than a reference value (and the like) indicates a value that is increased from the reference value by 30% or more.

Also as used herein, unless otherwise limited or defined, "integral" and derivatives thereof (e.g., "integrally") describe elements that are manufactured as a single piece without fasteners, adhesive, or the like to secure separate components together. For example, an element stamped, cast, or otherwise molded as a single-piece component from a single piece of sheet metal or using a single mold, without rivets, screws, or adhesive to hold separately formed pieces together is an integral (and integrally formed) element. In contrast, an element formed from multiple pieces that are separately formed initially then later connected together, is not an integral (or integrally formed) element.

Before we go on to set out the claims, we first set out the following clauses describing some prominent features of certain embodiments of the invention:
*1. An endotracheal tube holder device, comprising:*
   *a head strap;*
   *a holding body including a first cheek bracket and a second cheek bracket connected by a bar, the first cheek bracket including a first strap loop and a second strap loop, and the second cheek bracket including a third strap loop and a fourth strap loop;*
   *a tube holder secured to the holding body and configured to secure an endotracheal tube,*
   *wherein the first strap loop, the second strap loop, the third strap loop, and the fourth strap loop are configured to secure the head strap to the holding body.*
*2. The endotracheal tube holder device of clause 1, wherein the holding body further includes a first pad and a second pad that are configured to secure to a patients face.*
*3. The endotracheal tube holder device of clause 2, wherein the first pad and the second pad are transparent or translucent.*
*4. The endotracheal tube holder device of any one of clauses 1 to 3, wherein the first strap loop comprises a first axis, the second strap loop comprises a second axis, the third strap loop comprises a third axis, and the fourth strap loop comprises a fourth axis.*
*5. The endotracheal tube holder device of clause 4, wherein the first axis and the second axis form an angle that is between about 95° and about 170°.*
*6. The endotracheal tube holder device of any one of clauses 1 to 5, wherein the tube holder includes a tube holder body and a plurality of teeth extending from a bottom surface of the tube holder body.*
*7. The endotracheal tube holder device of clause 6, wherein each of the plurality of teeth define an axis, and*
   *wherein each of the axes of the plurality of teeth are substantially perpendicular to an outer surface of the endotracheal tube when the endotracheal tube is attached to the endotracheal tube holder device.*
*8. The endotracheal tube holder device of any one of clauses 1 to 7, wherein the tube holder comprises a bite block.*
*9. A system, comprising:*
   *a holding body;*
   *a head strap secured to the holding body at four points of contact;*
   *a tube holder secured with the holding body, the tube holder including a tube holder body and a plurality of teeth extending from a bottom surface of the tube holder body, and the plurality of teeth including a plurality of first teeth and a plurality of second teeth,*
   *wherein a distance between the bottom surface of the tube holder body and a tip of one of the plurality of first teeth is larger than a distance between the bottom surface of the tube holder body and a tip of one of the plurality of second teeth.*
*10. The system of clause 9, wherein the holding body further includes a first pad and a second pad that are configured to secure to a patients face.*
*11. The system of clause 10, wherein the first pad and the second pad are transparent or translucent.*
*12. The system of any one of clauses 9 to 11, wherein the holding body includes a first cheek bracket and a second cheek bracket,*
   *wherein the first cheek bracket includes a first strap loop and a second strap loop, and the second cheek bracket includes a third strap loop and a fourth strap loop, and*
   *wherein the first strap loop, the second strap loop, the third strap loop, and the fourth strap loop define the four points of contact with the head strap.*
*13. The system of any one of clauses 9 to 12, wherein the bottom surface of the tube holder body defines a longitudinal center plane that is normal to the bottom surface, and*
   *wherein each of the plurality of teeth comprise an axis that is angled with respect to the longitudinal center plane.*
*14. The system of any one of clauses 9 to 13, wherein a tube strap is secured with the tube holder and configured to wrap around an endotracheal tube, and*
   *wherein the tube strap comprises a plurality of ribs thereon.*
*15. The system of any one of clauses 9 to 14, wherein the plurality of teeth are aligned in four columns, and*
   *wherein the plurality of teeth in one of the four columns converge toward the plurality of teeth in a different column below the bottom surface.*
*16. The system of any one of clauses 9 to 15, wherein the endotracheal tube holder device includes more second teeth than first teeth.*
*17. A system, comprising:*
   *a holding body including a first pad and a second pad, the first pad and the second pad are transparent or translucent; and*
   *a tube holder secured with the holding body, the tube holder includes a tube holder body and a plurality of teeth extending from a bottom surface of the tube holder body,*
   *wherein the bottom surface of the tube holder body defines a longitudinal center plane that is normal to the bottom surface, and*
   *wherein each of the plurality of teeth comprise an axis that is not parallel with the longitudinal center plane.*
*18. The system of clause 17, wherein each of the axes of the plurality of teeth converge toward the longitudinal center plane below the bottom surface.*
*19. The system of clause 17 or 18, further comprising a head strap secured to the holding body at four points of contact.*
*20. The system of any one of clauses 17 to 19, wherein a tube strap is secured with the tube holder and configured to wrap around an endotracheal tube, and*
   *wherein the tube strap comprises a plurality of ribs thereon.*

Various features and advantages of the disclosure are set forth in the following claims.

## Claims

1. A system (100), comprising:
a holding body (110);
a head strap (106) secured to the holding body (110) at four points of contact; and
a tube holder (118) secured with the holding body (110), the tube holder (118) including a tube holder body (202) and a plurality of teeth (310) extending from a bottom surface (312) of the tube holder body (202), and the plurality of teeth (310) including a plurality of first teeth (314) and a plurality of second teeth (316),
wherein a distance (D3) between the bottom surface (312) of the tube holder body (202) and a tip of one of the plurality of first teeth (314) is larger than a distance (D4) between the bottom surface (312) of the tube holder body (202) and a tip of one of the plurality of second teeth (316).

2. The system (100) of claim 1, wherein the holding body (110) further includes a first pad (114) and a second pad (116) that are configured to secure to a patient's face.

3. The system (100) of claim 2, wherein the first pad (114) and the second pad (116) are transparent or translucent.

4. The system (100) of any one of claims 1 to 3, wherein the holding body (110) includes a first cheek bracket (132) and a second cheek bracket (134),
wherein the first cheek bracket (132) includes a first strap loop (174) and a second strap loop (176), and the second cheek bracket (134) includes a third strap loop (174, 176) and a fourth strap loop (174, 176), and
wherein the first strap loop (174), the second strap loop (176), the third strap loop (174, 176), and the fourth strap loop (174, 176) define the four points of contact with the head strap (106).

5. The system (100) of claim 4, wherein the first strap loop (174) comprises a first axis (A1), the second strap loop (176) comprises a second axis (A2), the third strap loop (174, 176) comprises a third axis (A3), and the fourth strap loop (174, 176) comprises a fourth axis (A4).

6. The system (100) of claim 5, wherein the first axis (A1) and the second axis (A2) form an angle (B1) that is between about 95° and about 170°.

7. The system (100) of claims 5 or 6, wherein the third axis (A3) and the fourth axis (A4) form an angle (B2) that is between about 95° and about 170°.

8. The system (100) of any one of claims 5 to 7, wherein the first axis (A1) and the fourth axis (A4) are substantially perpendicular to a bar (130) of the holding body (110).

9. The system (100) of any one of the preceding claims, wherein the bottom surface (312) of the tube holder body (202) defines a longitudinal center plane (AC) that is normal to the bottom surface (312), and
wherein each of the plurality of teeth (310) comprise an axis that is angled with respect to the longitudinal center plane (AC).

10. The system (100) of any one of the preceding claims, wherein a tube strap (120) is secured with the tube holder (118) and configured to wrap around an endotracheal tube (102), and
wherein the tube strap (120) comprises a plurality of ribs (214) thereon.

11. The system (100) of any one of the preceding claims, wherein the plurality of teeth (310) are aligned in four columns (C1, C2, C3, C4), and
wherein the plurality of teeth (310) in one of the four columns (C1, C2, C3, C4) converge toward the plurality of teeth (310) in a different column (C1, C2, C3, C4) below the bottom surface (312).

12. The system (100) of any one of the preceding claims, wherein the tube holder (118) includes more of the second teeth (316) than the first teeth (314).

13. The system (100) of any one of the preceding claims, wherein the bottom surface (312) of the tube holder body (202) defines a longitudinal center plane (AC) that is normal to the bottom surface (312), and
wherein each of the plurality of teeth (310) comprises an axis that converges toward the longitudinal center plane (AC) below the bottom surface (312).

14. The system (100) of any one of the preceding claims, wherein each of the plurality of teeth (310) defines an axis, and
wherein each of the axes of the plurality of teeth (310) is substantially perpendicular to an outer surface (340) of an endotracheal tube (102) when the endotracheal tube (102) is attached to the system (100).

15. The system (100) of any one of the preceding claims, wherein the tube holder (118) comprises a bite block (204).
